# EUROPEAN PATENT APPLICATION

(11) **EP 2 810 594 A1**
(43) Date of publication of application: **10.12.2014**
(21) Application number: 13744113.5
(22) Date of filing: 01.02.2013
(51) Int. Cl.: A47L 15/42, A47L 15/44, D06F 37/28, D06F 39/02

(54) **WASHING DEVICE**

(30) Priority: 02.02.2012 JP 2012021371
(71) Applicant: Saraya Co., Ltd., Osaka-shi Osaka 546-0013 (JP); Neosys Corporation, Saitama 350-2287 (JP)
(72) Inventor: SHINTANI, Naoki, Osaka-shi Osaka 546-0013 (JP); KAMEDA, Takeshi, Osaka-shi Osaka 546-0013 (JP); YOSHIJIMA, Tomohiro, Tsurugashima-shi Saitama 350-2287 (JP)
(74) Representative: Hering, Hartmut
(86) International application number: PCT/JP2013/052424
(87) International publication number: WO 2013/115392

(57) **Abstract**

A washing device is provided which reduces a load on a user while ensuring favorable operability and safety. An opening/closing door (2) includes, at a front surface thereof, a control panel (3) which includes at least a setting portion (4) and a display portion (5), a handle portion (8) for operating a holding portion used in opening/closing the opening/closing door (2), a cover (7) which covers the handle portion (8) during washing of a to-be-washed object, and first detection means which detects movement of the cover (7). The cover (7) is provided so as to be movable between the handle portion (8) and the control panel (3). By moving the cover (7) during washing of the to-be-washed object, the cover (7) causes the handle portion (8) to appear on the front surface of the opening/closing door (2) to enable an operation of the holding portion and disables an operation of the control panel (3) by covering the control panel (3). The first detection means temporarily stops scattering of washing water to cause a standby state.

## Description

### TECHNICAL FIELD

The present invention relates to a washing device. More specifically, the present invention relates to a washing device including: a washing tank in which a to-be-washed object is washed; a washing water supply portion which supplies washing water to the washing tank; a washing nozzle which performs washing by scattering the washing water to the to-be-washed object within the washing tank; and a casing which houses all of these components and includes an opening/closing door through which at least the to-be-washed object is taken in and out.

### BACKGROUND ART

Conventionally, for example, the technique described in Japanese Examined Utility Model Publication No. 53-13234 is known as such a washing device as described above.

In Patent Document 1, it is configured that prior to opening of a door, an opening/closing detection switch is turned off and a washing operation is stopped. An operation of turning off the switch and opening of the door are performed during one operation. Therefore, when an operation lever is quickly operated, there is the possibility that the opening/closing detection switch is operated to be turned off and the door is opened before spraying of washing water is completely stopped, and thus the washing water scatters to the outside.

As a countermeasure against this, it is conceivable that the opening/closing detection switch is operated with a dedicated safety lever independently of an operation of opening the door. However, since two operations of the safety lever and the operation lever are required, the operation is very complicated, and thus there is the possibility that the operability is deteriorated.

### CITATION LIST

### [PATENT DOCUMENTS]

[PATENT DOCUMENT 1] Japanese Examined Utility Model Publication No. 53-13234

### SUMMARY OF THE INVENTION

### PROBLEMS TO BE SOLVED BY THE INVENTION

Therefore, an object of the present invention is to provide a washing device which is able to prevent washing water from scattering to the outside when a door is opened, while ensuring favorable operability.

### SOLUTION TO THE PROBLEMS

In order to achieve the above-described object, a feature of a washing device according to the present invention is that in a configuration of including: a washing tank in which a to-be-washed object is washed; a washing water supply portion which supplies washing water to the washing tank; a washing nozzle which performs washing by scattering the washing water to the to-be-washed object within the washing tank; and a casing which houses all of these components and includes an opening/closing door through which at least the to-be-washed object is taken in and out, the opening/closing door includes, at a front surface thereof, a control panel which includes at least a setting portion and a display portion, a handle portion for operating a holding portion used in opening/closing the opening/closing door, a cover which covers the handle portion during washing of the to-be-washed object, and first detection means which detects movement of the cover, the cover is configured to be movable between the handle portion and the control panel, and by moving the cover during washing of the to-be-washed object, the cover causes the handle portion to appear on the front surface of the opening/closing door to enable an operation of the holding portion and also disables an operation of the control panel by covering the control panel, and the first detection means temporarily stops scattering of the washing water to cause a standby state.

According to the above configuration, when the user moves the cover covering the handle portion during washing in order to open the opening/closing door, the first detection means detects the movement of the cover and temporarily stops scattering of the washing water to cause the standby state. Then, as a result of the movement of the cover, the handle portion appears on the front surface of the opening/closing door to enable an operation of the holding portion, and thus the opening/closing door is made openable. As described above, it is not possible to open the door unless at least an operation (moving) of the cover and an operation of the holding portion are performed, and thus due to a time difference between these operations, it is possible to completely stop scattering (spraying) of the washing water when the opening/closing door is opened, and it is possible to prevent the washing water from scattering to the outside. In addition, since the cover is movable between the handle portion and the control panel, the moved cover covers the control panel. Thus, the user cannot operate the control panel to release the standby state. Therefore, it is possible to also prevent change, completion, or the like of a washing state caused due to an erroneous operation and to maintain the washing state. Moreover, it is possible to perform the above-described stop of spraying of the washing water and opening of the opening/closing door from moving of the cover to an operation of the holding portion by a continuous motion, and thus the operability is also improved.

In addition, in order to achieve the above-described object, another feature of the washing device according to the present invention is that in a configuration of including: a washing tank in which a to-be-washed object is washed; a washing water supply portion which supplies washing water to the washing tank; a washing nozzle which performs washing by scattering the washing water to the to-be-washed object within the washing tank; and a casing which houses all of these components and includes an opening/closing door through which at least the to-be-washed object is taken in and out, the opening/closing door includes, at a front surface thereof, a control panel which includes at least a setting portion and a display portion, a handle portion for operating a holding portion used in opening/closing the opening/closing door, a cover which covers the handle portion during washing of the to-be-washed object, and first detection means which detects movement of the cover, and by moving the cover during washing of the to-be-washed object, the cover causes the handle portion to appear on the front surface of the opening/closing door to enable an operation of the holding portion, and the first detection means temporarily stops scattering of the washing water to cause a standby state and disables an operation of the control panel.

According to the above configuration, when the user moves the cover covering the handle portion during washing in order to open the opening/closing door, the first detection means temporarily stops scattering of the washing water to cause the standby state. Then, as a result of the movement of the cover, the handle portion appears on the front surface of the opening/closing door to enable an operation of the holding portion, and thus the opening/closing door is made openable. As described above, it is not possible to open the door unless at least an operation (moving) of the cover and an operation of the holding portion are performed, and thus due to a time difference between these operations, it is possible to completely stop scattering (spraying) of the washing water when the opening/closing door is opened, and it is possible to prevent the washing water from scattering to the outside. In addition, since the first detection means further disables an operation of the control panel, the user cannot operate the control panel to release the standby state. Thus, it is possible to also prevent change, completion, or the like of a washing state caused due to an erroneous operation and to maintain the washing state. Moreover, it is possible to perform the above-described stop of spraying of the washing water and opening of the opening/closing door from moving of the cover to an operation of the holding portion by a continuous motion, and thus the operability is also improved.

In each configuration described above, the handle portion may include second detection means which detects an operation of the holding portion, and the second detection means may reset the standby state to cause a complete stop state. Thus, washing is completely stopped when the holding portion is operated, and hence it is possible to prevent change, completion, or the like of a washing state caused due to an erroneous operation of the cover and to maintain the washing state.

In the configurations described above, the first detection means may disable an operation of the control panel by at least turning off power of the setting portion. In addition, the first detection means may cause the display portion to display a message of operation disablement to disable an operation of the control panel.

In each configuration described above, a container holding portion which holds a container containing a detergent may be provided at a front surface of the casing at which the opening/closing door is opened. Thus, container replacement is not possible during washing, and hence it is possible to prevent change, completion, or the like of a washing state caused due to maintenance and to maintain the washing state.

In each configuration described above, for example, the to-be-washed object is a medical instrument.

### ADVANTAGEOUS EFFECTS OF THE INVENTION

According to the above-described features of the washing device according to the present invention, it is possible to prevent the washing water from scattering to the outside when the door is opened, while ensuring favorable operability.

Other objects, configurations, and effects of the present invention will become apparent from the following description of embodiments of the invention.

### BRIEF DESCRIPTION OF THE DRAWINGS

[FIG. 1] FIG. 1 is a perspective view of a washing device according to the present invention.
[FIG. 2] FIG. 2 is a diagram of a state where an opening/closing door is opened.
[FIG. 3] FIG. 3 is a partially-cutaway perspective view of the washing device.
[FIG. 4] FIG. 4 is a diagram illustrating connection of each member.
[FIG. 5] FIG. 5 is a diagram showing a hardware configuration of the washing device.
[FIG. 6] FIG. 6 is a diagram illustrating a sliding cover stop mechanism.
[FIG. 7] FIG. 7 is a schematic front view illustrating a state where a sliding cover is mounted.
[FIG. 8] FIG. 8 is a schematic side view illustrating a door opening/closing mechanism.
[FIG. 9] FIG. 9 is a diagram showing a relation among the sliding cover, a handle portion, and first detection means.
[FIG. 10] FIG. 10 is a control flow diagram regarding door opening/closing in the present invention.
[FIG. 11] FIG. 11 is a cross-sectional view of a container-attaching structure when a container is attached.
[FIG. 12] FIG. 12 is a cross-sectional view of the container-attaching structure after the container is attached.
[FIG. 13] FIG. 13 is a diagram illustrating a state where a connection holder, a connection adapter, a container holding lever, and the container are engaged.
[FIG. 14] FIG. 14 is a central longitudinal cross-sectional view of FIG. 13.
[FIG. 15] FIG. 15 is a front view of the container-attaching structure.
[FIG. 16] FIG. 16 is a perspective view of the container-attaching structure.
[FIG. 17] FIG. 17 is a perspective cross-sectional view of the container-attaching structure at the time of attachment to the washing device.
[FIG. 18] FIG. 18 is a cross-sectional view of a container storing portion of a housing.
[FIG. 19] FIG. 19 is an exploded perspective view of each member of the container-attaching structure.

### DESCRIPTION OF EMBODIMENTS

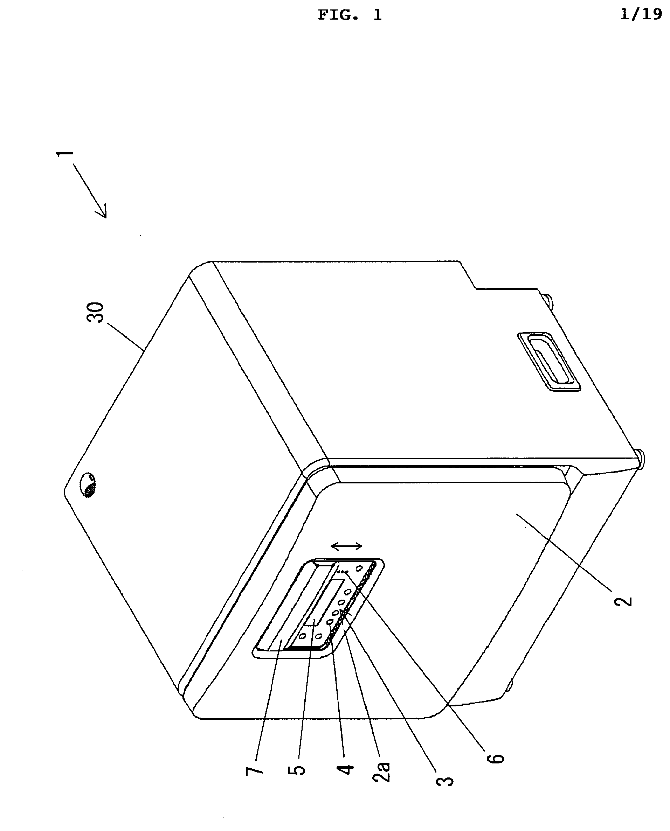

In FIG. 1, reference character 1 denotes a washing device main body, reference character 2 denotes an opening/closing door which is rotatably mounted on a casing 30 of the washing device 1, and reference character 3 denotes a control panel which is provided at a front surface of the opening/closing door 2. The control panel 3 includes: a setting portion 4 with which a user sets a washing process or the like when using the washing device 1; a display portion 5 which is provided near the setting portion 4, for displaying information in setting the washing process or the like, a state or error information during driving of the washing device 1, etc.; and a sound transmission portion 6 which gives a warning to the user by means of a sound, an alarm, a buzzer, or the like when an operation guide or abnormality of the device occurs. In addition, a handle portion 8 for operating a holding portion 32 used in opening/closing the opening/closing door 2 is provided on the front surface of the opening/closing door 2. The control panel 3 and the handle portion 8 are provided within a window portion 2a provided in the front surface of the opening/closing door 2, such that the control panel 3 and the handle portion 8 are aligned vertically.

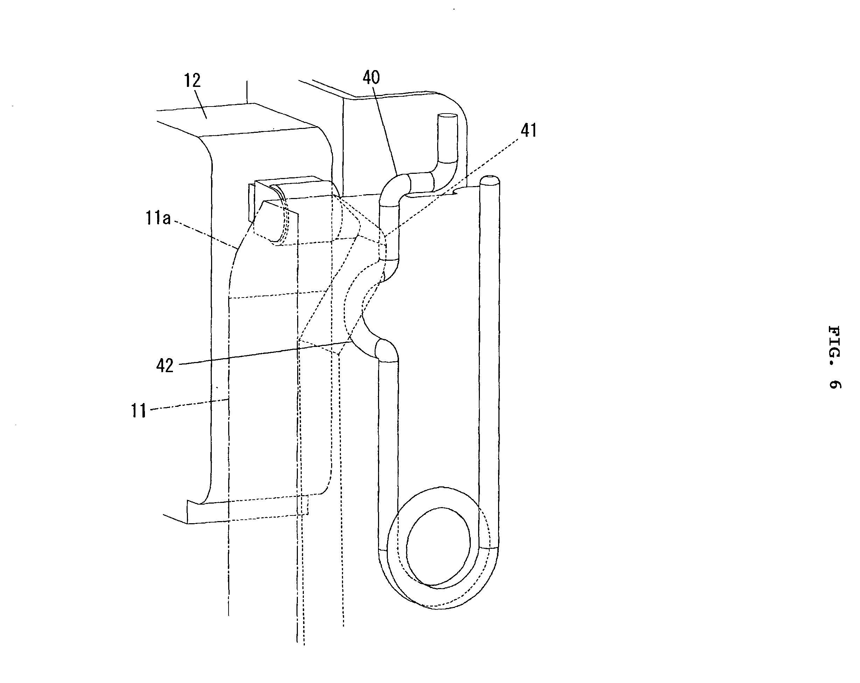

Reference character 7 denotes a sliding cover which is provided at the opening/closing door 2 and is slid so as to cover the control panel 3 in opening/closing the opening/closing door 2. As shown in FIGS. 1 and 6, the sliding cover 7 is provided in the window portion 2a so as to be slidable up and down between the control panel 3 and the handle portion 8.

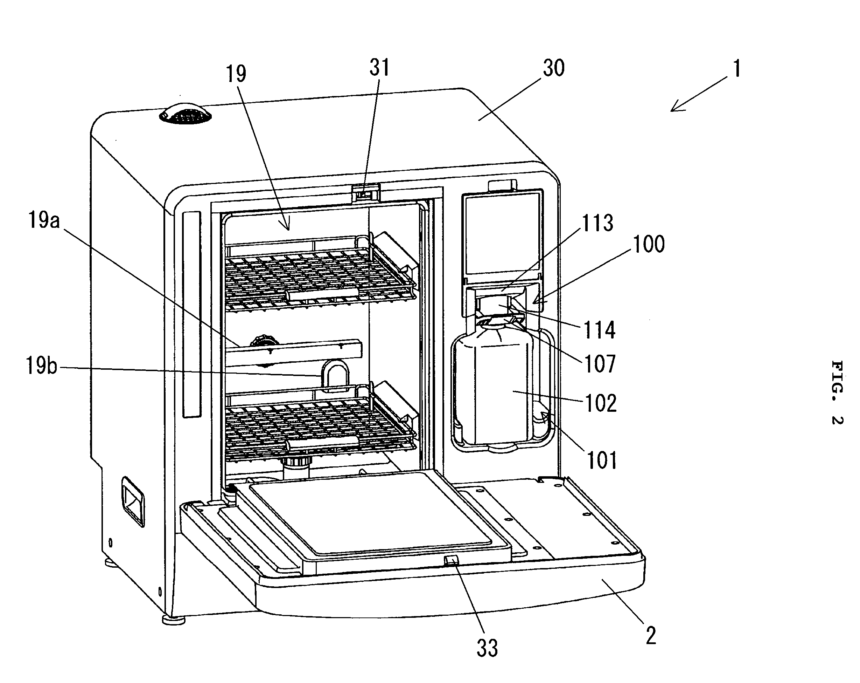

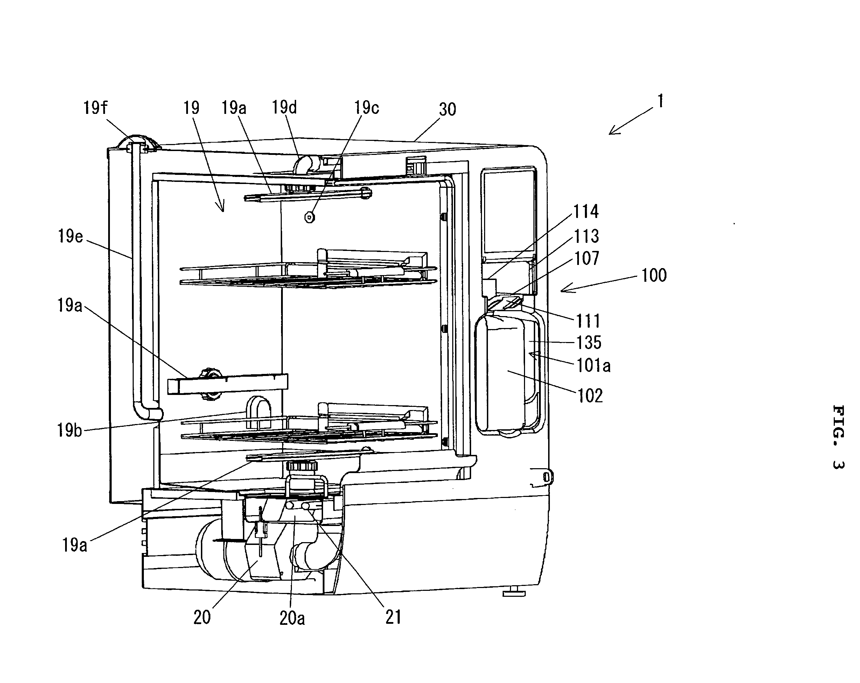

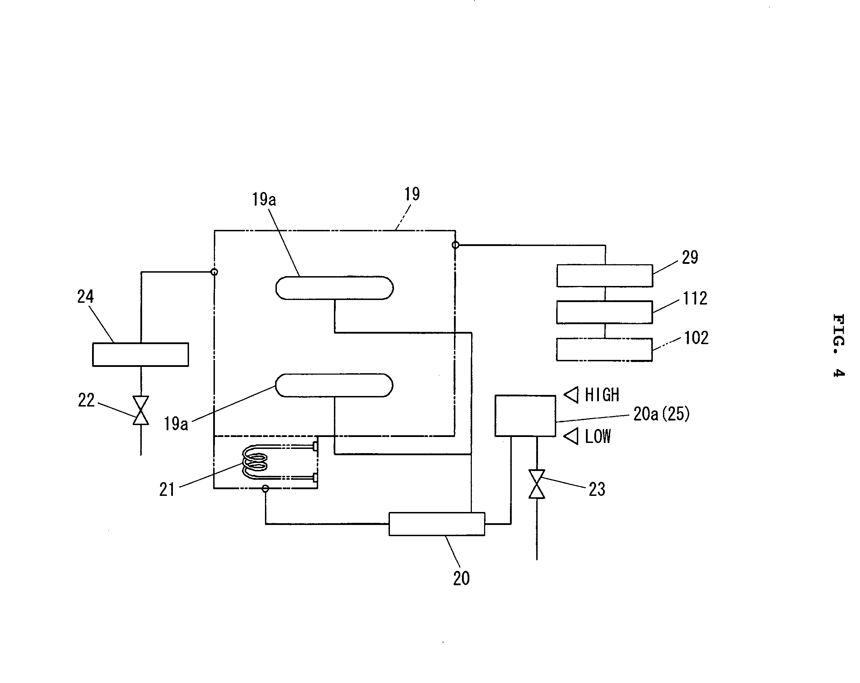

As shown in FIGS. 2 to 4, a washing tank 19 in which a to-be-washed object is washed and a container holding portion 100 which is located laterally to the washing tank 19 and holds a container 102 which contains a detergent are provided in the casing 30. The container holding portion 100 is provided at the front surface of the casing 30. Thus, it is not possible to replace the container 102 unless the opening/closing door 2 is opened, and container replacement during washing is prevented and an interruption of the washing process is prevented.

A washing water supply portion which supplies washing water to the washing tank 19, a detergent supply portion which supplies the detergent to the washing tank 19, three washing nozzles 19a which perform washing by scattering the washing water mixed with the detergent to the to-be-washed object within the washing tank 19, a washing water circulation portion which recirculates used washing water for washing, and a drain portion which drains the washing water are connected to the washing tank 19. The washing water supply portion includes a water supply valve 22 which adjusts an amount of water supplied from a water supply port and a water supply sensor 24 which detects an amount of supplied water, and supplies the washing water through the water supply port 19b into the washing tank 19. The detergent supply portion includes a detergent pump 112 and a detergent amount detection sensor 29, and supplies the detergent through a detergent supply port 19c into the washing tank 19. In addition, for example, a reversible pump is used as a washing water pressure pump 20 in the washing water circulation portion, and is utilized for circulating and draining the washing water. The pump 20 distributes the washing water through a pipe 19d to the washing nozzles 19a. The drain portion is provided with a storage tank 20a connected to the reversible pump 20 and a drain valve 23 which adjusts an amount of water drained, and monitors a water level within the storage tank 20a with a water level detection sensor 25. It should be noted that a pipe 19e and a discharge portion 19f through which steam within the washing tank 19 is discharged are provided to the washing tank 19. In addition, a tank internal temperature sensor 26, a strainer detection sensor 27, and a washing nozzle rotation detection sensor 28 for confirming a bubbling state from a driven state of the pump and a rotation speed of each washing nozzle are provided thereto.

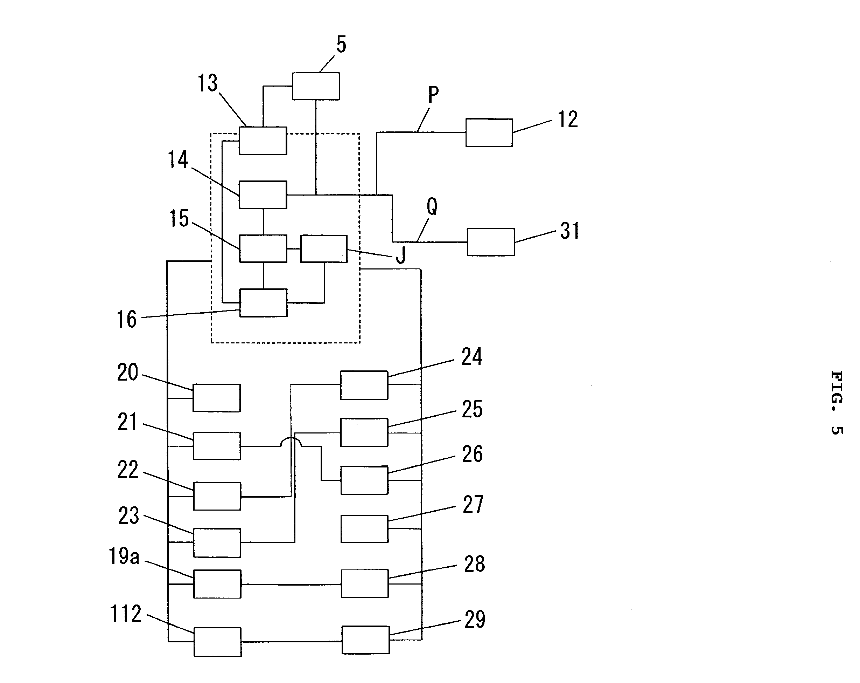

Here, FIG. 5 shows a hardware configuration of the washing device. A control unit 13 accepts an input from the setting portion 4 and executes a program J, and also controls the washing nozzles 19a, the reversible pump 20, a heater 21, the water supply valve 22, the drain valve 23, the detergent supply pump 112, and the like. The control unit 13 includes a determination section 14 which determines an opened/closed state of the opening/closing door 2 on the basis of signals P and Q from later-described first and second detection means 12 and 31; a memory 15; and a maintenance management memory 16. In addition, the control unit 13 performs the above control on the basis of inputs from the determination section 14 and the various sensors 24 to 29, and also controls the display portion 5 and the sound transmission portion 6.

A sliding cover stop mechanism 35 which locks the sliding cover 7 above the control panel 3 or the handle portion 8 is provided at the sliding cover 7. As shown in FIG. 6, the sliding cover stop mechanism 35 includes: a sliding cover projection portion 11 which is provided at a side portion of the sliding cover 7; and a sliding cover spring 40 which is provided so as to be opposed to the projection portion 11. The sliding cover projection portion 11 includes: a contact surface 11a which contacts the later-described cover micro switch 12; and a sliding restriction projection portion 41 which restricts sliding of the sliding cover 7. The sliding cover spring 40 deforms when a force applied thereto is great, and returns to the original state when the force is eliminated. The sliding cover spring 40 includes a spring projection portion 42 which projects toward the sliding cover projection portion 11. As shown in FIG. 6, owing to the mechanism 35, the sliding cover projection portion 11 is urged upward by the sliding cover spring 40 and the sliding restriction projection portion 41 and the spring projection portion 42 are engaged with each other, whereby the sliding cover 7 is held in a state of covering the handle portion 8 and the contact surface 11a contacts the switch 12. When the sliding cover 7 is slid downward, the sliding cover spring 40 deforms and the engagement between the sliding restriction projection portion 41 and the spring projection portion 42 is released. In addition, with the release of the engagement, the contact surface 11 a is separated from the switch 12, and the movement of the sliding cover 7 is detected. Then, the sliding cover 7 causes the handle portion 8 to appear on the front surface of the opening/closing door 2 and also covers the control panel 3.

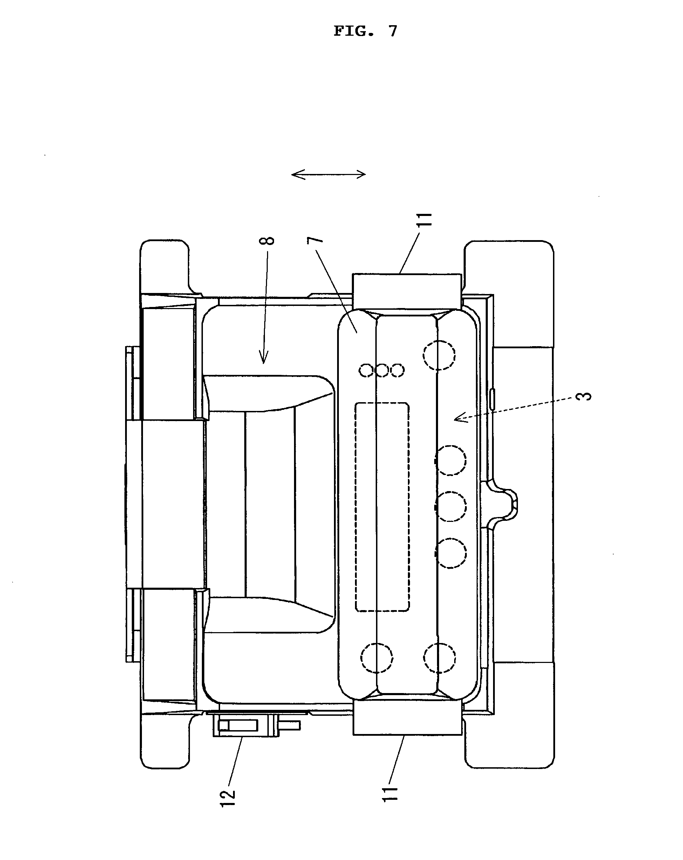

In addition, as shown in FIG. 7, the cover micro switch 12 as the first detection means which detects movement of the sliding cover 7 is provided within the opening/closing door 2 so as to contact the sliding cover projection portion 11. Only when the contact with the sliding cover projection portion 11 is released, the switch 12 is energized to transmit a sliding cover opening signal P to the determination section 14. As shown in FIG. 1, when the sliding cover 7 is located so as to provide a state where it is possible to operate the control panel 3 (the sliding cover 7 is located above the handle portion 8), if the sliding cover 7 even slightly moves from this position, a standby mode is activated.

As described above, in order to open the opening/closing door 2, the sliding cover 7 has to be moved. When the sliding cover 7 is moved, the sliding cover 7 covers the setting portion 4 as shown in FIG. 6. Thus, it is possible to physically prevent an erroneous operation from being performed in opening the opening/closing door 2. In addition, since the standby mode is activated on the basis of the sliding cover opening signal P from the switch 12, scattering of the washing water to the outside is prevented. On the other hand, in order to operate the setting portion 4, the user has to move the sliding cover 7 again. The sliding cover 7 that has been moved again covers the handle portion 8 as shown in FIG. 1, and thus the user cannot operate the holding portion 32 and cannot open the door. Therefore, it is possible to prevent an erroneous operation of the user and it is possible to safely perform an operation. It should be noted that a pair of the mechanisms 35 are proved at both side portions of the sliding cover 7.

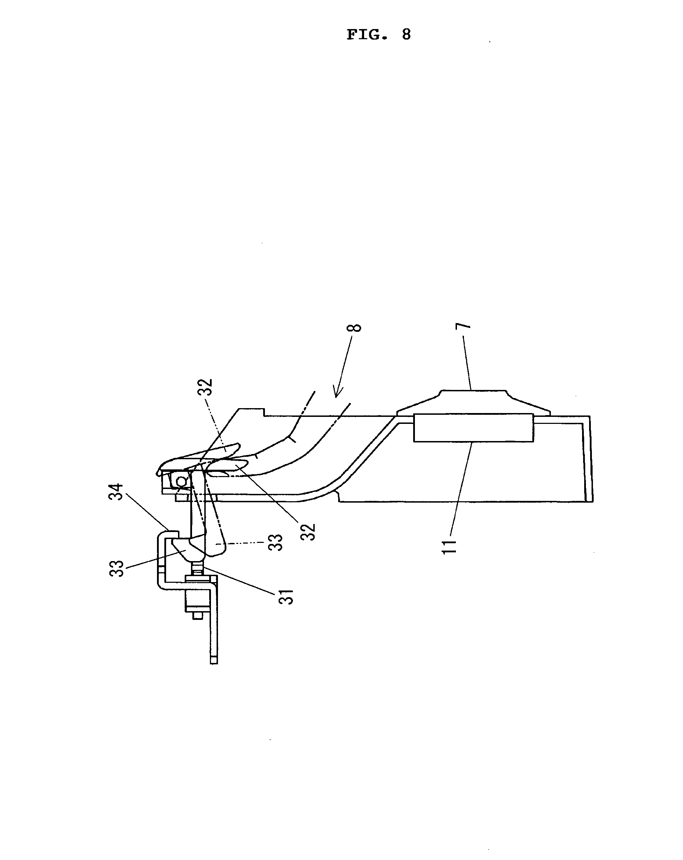

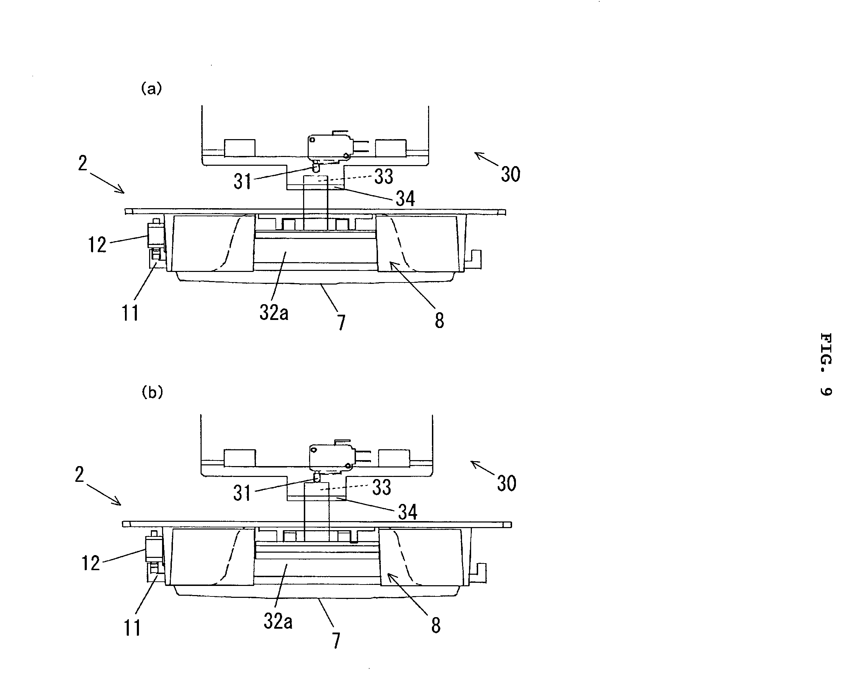

As shown in FIGS. 8 and 9, the handle portion 8 includes a holding mechanism which holds the opening/closing door 2 in a state where the opening/closing door 2 is closed. The holding mechanism includes at least a latch 33 and a latch hook portion 34 which is provided within a frame and at which the latch 33 is hooked, and is able to release a holding state by the holding portion 32 including a latch lever 32a which slides in conjunction with the latch 33. In this latch structure, the holding portion 32 for releasing holding by the latch 33 serves as the latch lever 32a. The latch 33 is released by gripping the holding portion 32 provided to the handle portion 8, whereby it is made possible to open the opening/closing door 2.

In addition, in the casing 30 which houses the washing tank 19, the casing micro switch 31 as the second detection means which detects an operation of the holding portion 32 is provided such that the casing micro switch 31 contacts the latch 33 when the opening/closing door 2 is closed. Only when the opening/closing door 2 is opened and the contact between the casing micro switch 31 and the latch 33 is released, the switch 31 is energized to transmit an opening/closing door opening signal Q to the determination section 14. As shown by an alternate long and two short dashes line in FIG. 8, if the holding portion 32 is operated by the user and the opening/closing door 2 is even slightly opened, a complete stop mode is activated.

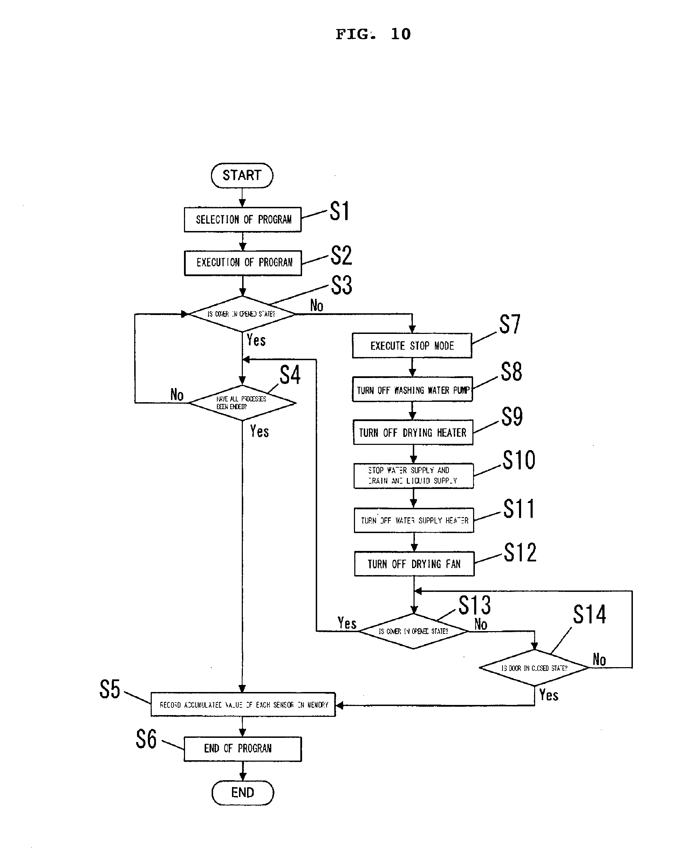

Here, a processing process performed with movement of the sliding cover 7 and an operation of the holding portion 32 during washing will be described with reference to FIG. 10.

First, when the user performs selection and setting of a program on the control panel 3 (step S1), the control unit 13 executes the program J (step S2). During washing, the sliding cover 7 is in a "closed state" where the sliding cover 7 covers the handle portion 8 and the opening/closing door 2 is in a "closed state" where the opening/closing door 2 is closed, and thus the sliding cover opening signal P and the opening/closing door opening signal Q are not transmitted from the cover micro switch 12 and the casing micro switch 31. Here, when the sliding cover 7 is moved, the cover micro switch 12 transmits the sliding cover opening signal P to the determination section 14. The determination section 14 determines whether the sliding cover 7 has been moved (the opened/closed state thereof), on the basis of presence/absence of the sliding cover opening signal P (step S3).

If the sliding cover opening signal P has not been received (the sliding cover 7 is in the "closed state", Yes in step S3), the control unit 13 continues to execute the program. Then, the control unit 13 determines whether all processes have been ended (step S4). If all the processes have been ended, the control unit 13 additionally writes a record of the processes performed so far and a driving time for which driving has been performed in each mode as an accumulated time for maintenance on the maintenance management memory 16 (step S5) and ends the execution of the program (step S6). On the other hand, if not all the processes have been ended, the control unit 13 continues to execute the program and the processing returns to the above-described step S3.

On the other hand, if the sliding cover opening signal P has been received (the sliding cover 7 is in an "opened state" where the sliding cover 7 covers the control panel 3, No in step S3), the control unit 13 switches the program to the standby mode (step S7). In addition, the processes performed so far and a driving time for which driving has been performed in each mode are recorded on the memory 15 of the control unit 13. Then, stop of the washing water pressure pump 20 (step S8), stop of the drying heater 21 (step S9), stop of water supply and drain (step S10), stop of a water supply heater (step S11), and stop of a drying fan (step S12) are sequentially performed. As described above, when the program being executed is merely stopped once, the contents thereof are stored in the memory 15, and the program is kept in a state where the program can be restarted.

Then, the determination section 14 determines again whether the sliding cover 7 has been moved (step S13). Here, if the sliding cover opening signal P has not been received, the control unit 13 restarts the program that has been executed, and the processing proceeds to the above-described step S4. Thus, even when the sliding cover 7 is moved by mistake, thereby causing the standby mode, it is possible to immediately return to the process that has been executed.

On the other hand, if the sliding cover opening signal P has been received, the opened/closed state of the opening/closing door 2 is determined. Here, when the opening/closing door 2 is opened, the casing micro switch 31 transmits the opening/closing door opening signal Q to the determination section 14. The determination section 14 determines the opened/closed state of the opening/closing door 2 on the basis of presence/absence of the opening/closing door opening signal Q (step S14).

If the opening/closing door opening signal Q has not been received (the opening/closing door 2 is in the "closed state", No in step S 14), the processing returns to step S13. On the other hand, if the opening/closing door opening signal Q has been received (the opening/closing door 2 is in an "opened state", Yes in step S14), the control unit 13 cancels the standby mode and switches to the complete stop mode, deletes the process management contents that have been temporarily recorded on the memory 15 in the standby mode, and additionally writes a record of the processes performed so far and a driving time for which driving has been performed in each mode as an accumulated time for maintenance on the maintenance management memory 16 (step S5). Thus, even when the opening/closing door 2 is closed again and the determination section 14 determines that the opening/closing door 2 is in the "closed state", since the contents that have been recorded on the memory 15 have been deleted, the program that has been executed before stop is not continuously executed. As in the present embodiment, when the to-be-washed object is a medical instrument, hygiene management by washing is guaranteed.

It should be noted that the standby mode is executed not only when the determination section 14 determines temporary stop of the device with opening/closing of the sliding cover 7 but also when an abnormal signal has been received from each of the above-described various sensors. Thus, this is effective means which minimizes trouble regarding the washing device. In this case, the content of the abnormality is displayed on the display portion 5.

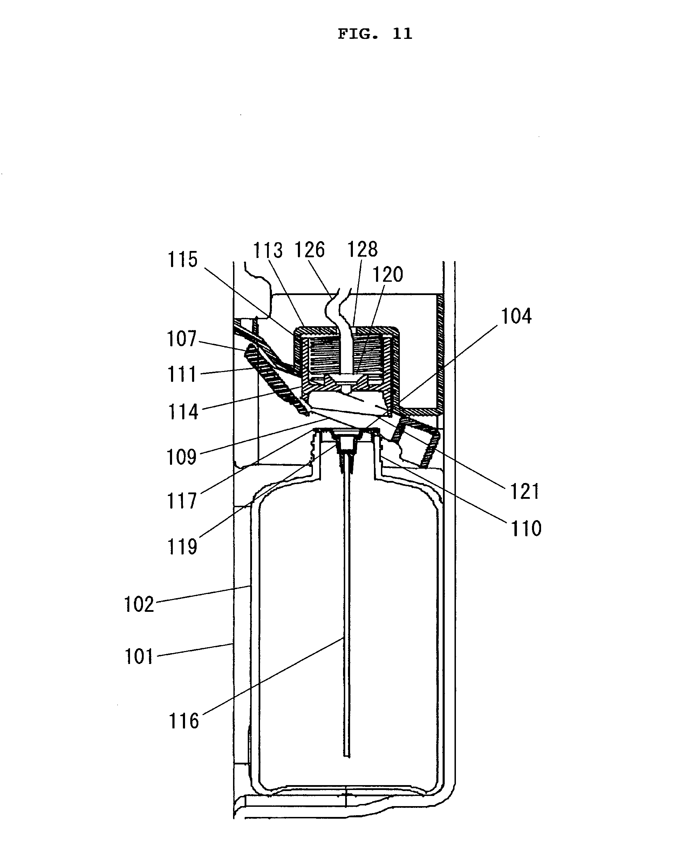

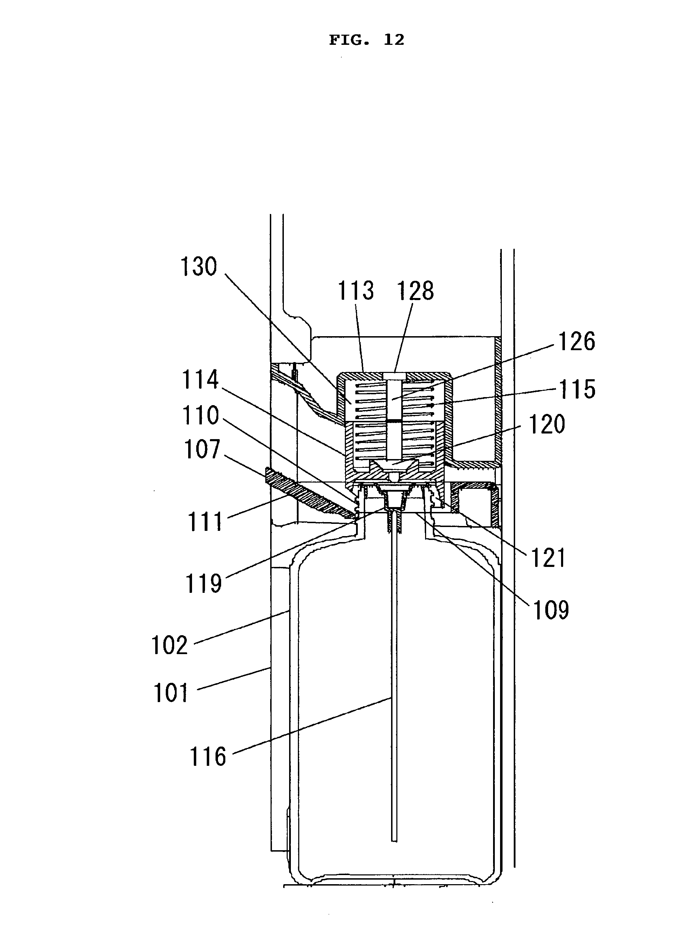

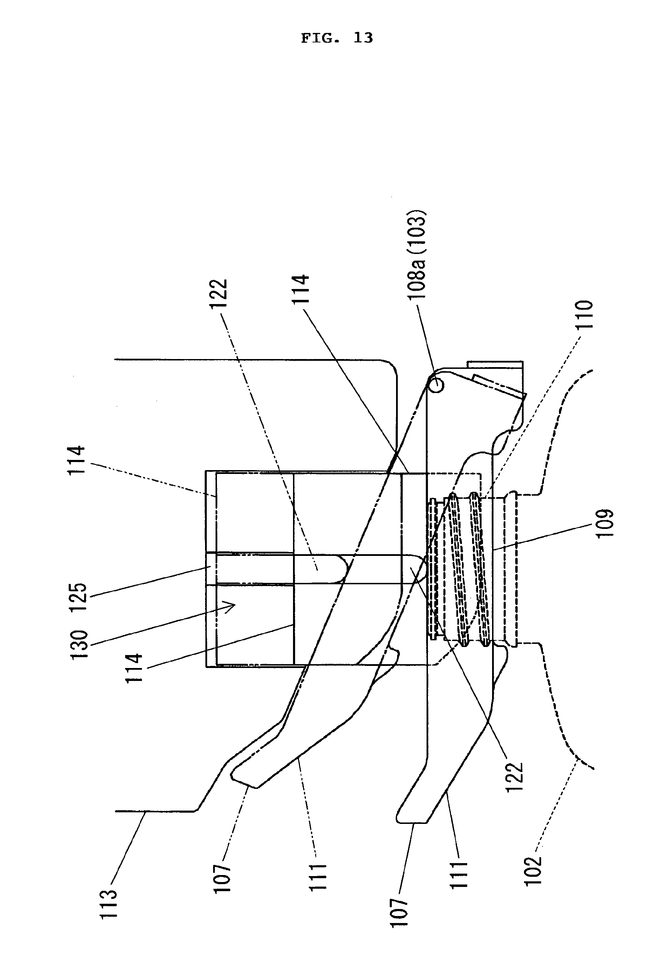

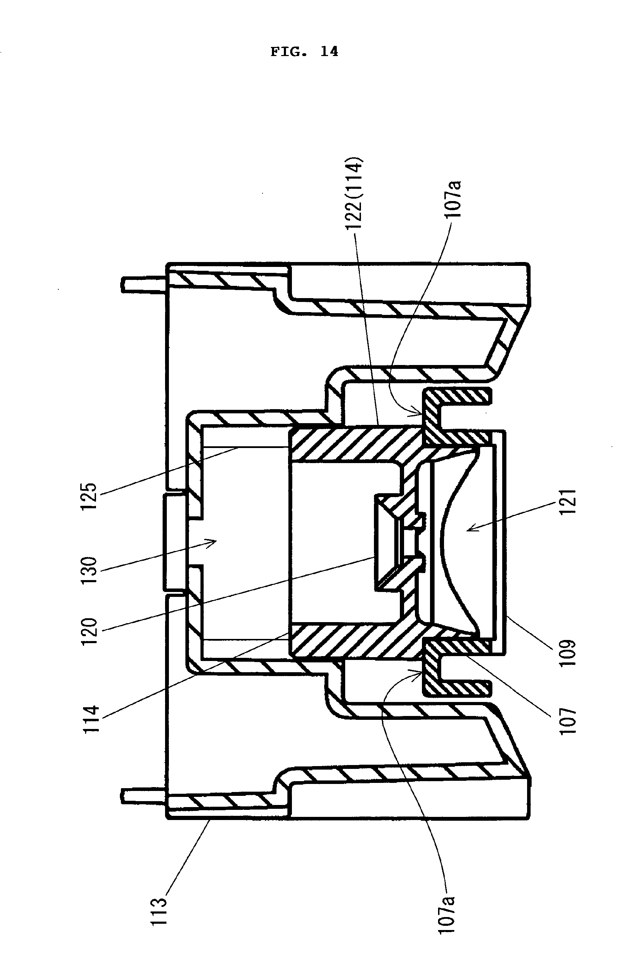

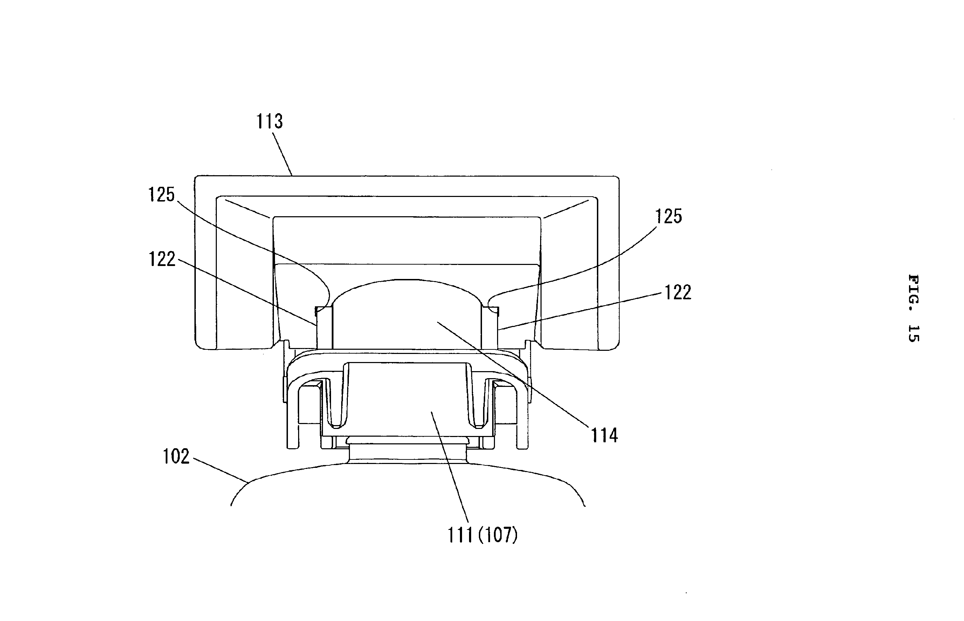

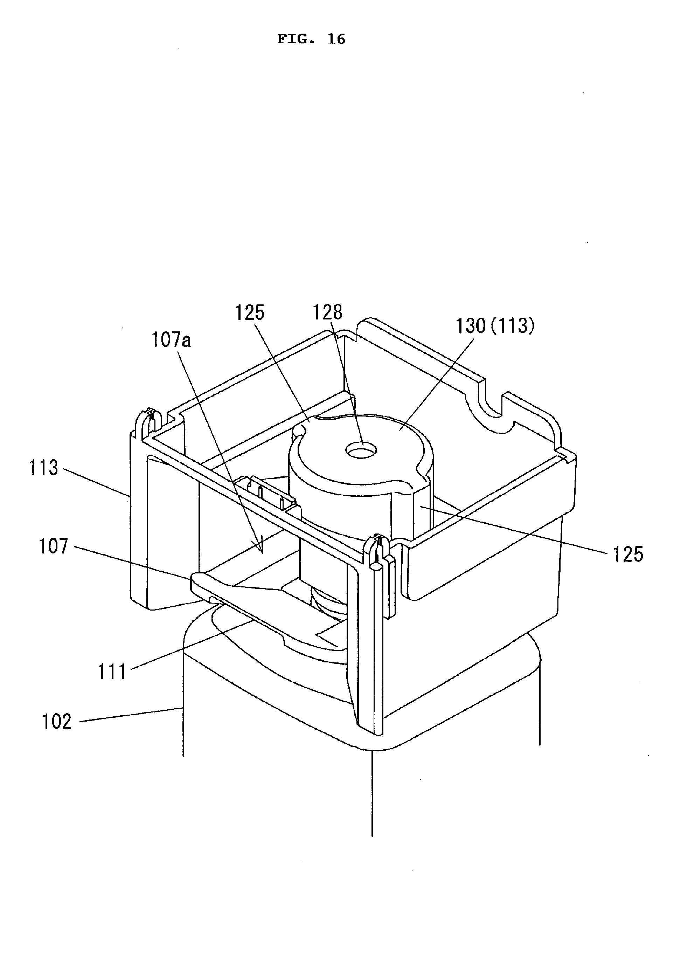

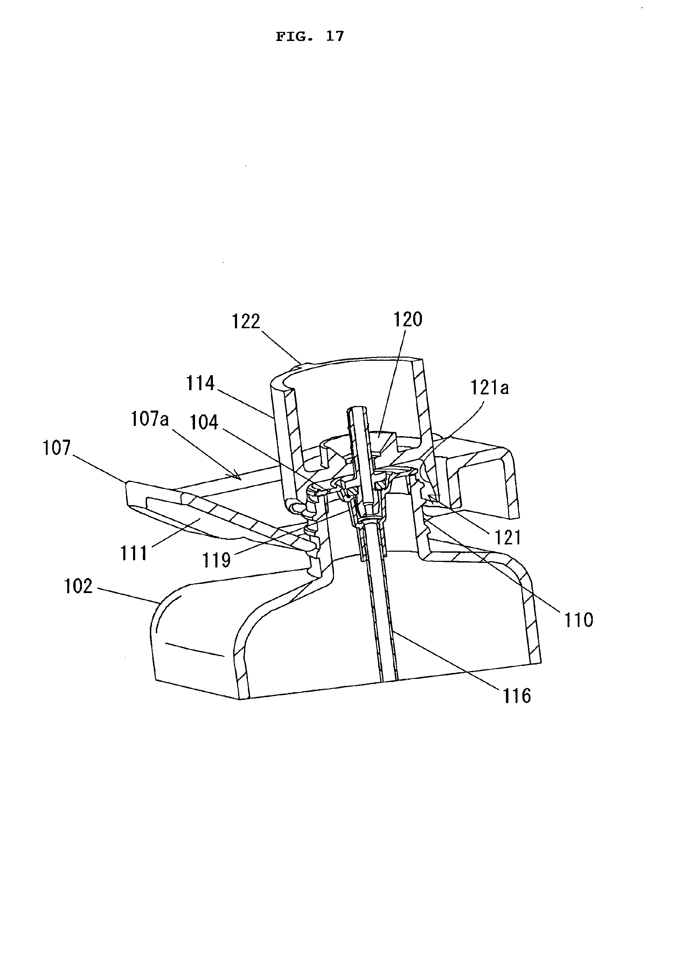

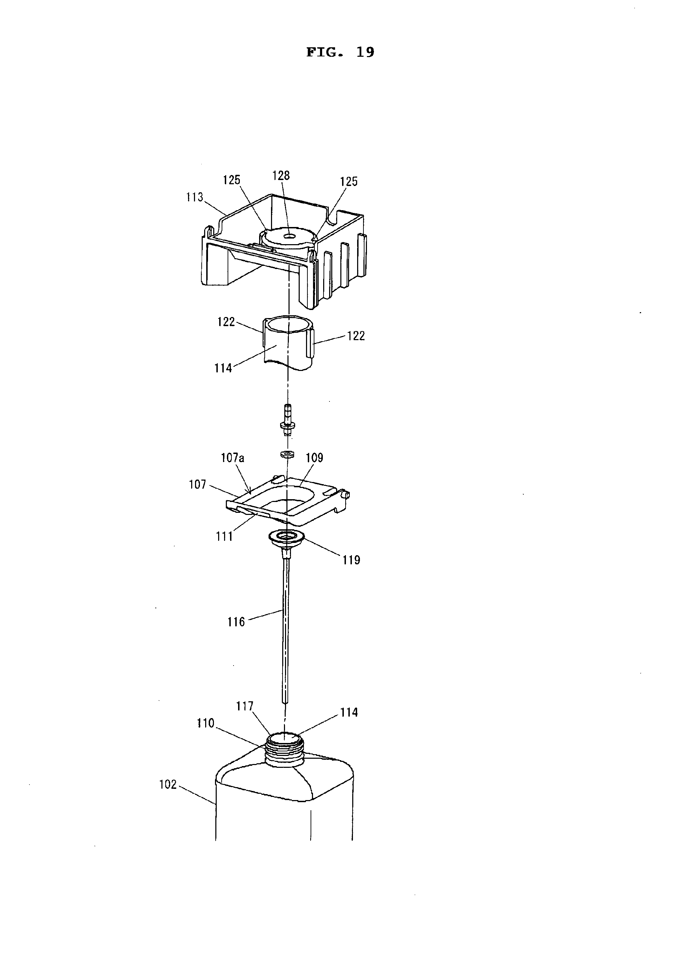

Next, an attaching/detaching mechanism for the detergent container used in the washing device 1 will be described below with reference to FIGS. 11 to 19.

The container holding portion 100 as the attaching/detaching mechanism for the container includes: a housing 101 which includes a storing portion 101a which stores the container 102; a spring 115 which holds the container 102 by urging the container 102 downward when the container 102 is attached; a container holding lever 107 which releases holding of the container 102 in detaching the container 102 by being moved upward; a connection holder 113 which is connected to the detergent pump 112 fixed to the housing 101; and a connection adapter 114 which is mounted so as to be slidable between the container holding lever 107 and the connection holder 113 in the up-down direction in attaching/detaching the container 102 and which is connected to the container 102.

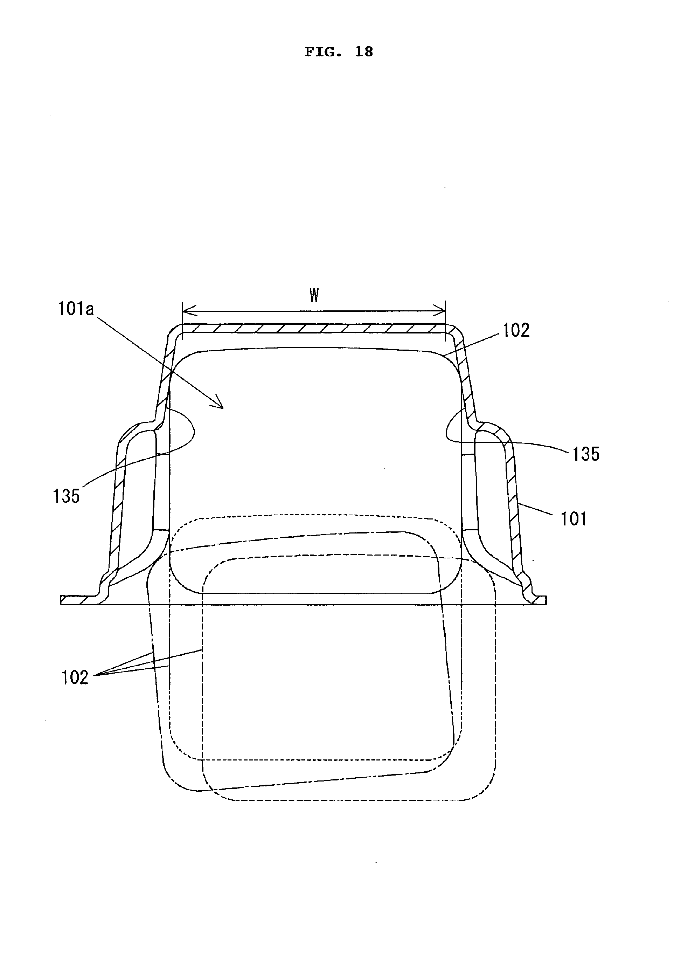

As shown in FIG. 18, an inner side wall 135 of the housing 101 is structured so as to be narrowed toward the back of the housing 101. In addition, the width W of the narrowest portion thereof is equal to or smaller than the width of the container 102. When the container 102 is pressed to the back of the housing 101, the container 102 is positioned at the center of the storing portion 101a of the housing 101 such that a through hole 109 of the container holding lever 107 and the connection adapter 114 are positioned so as to be concentric with the center. Moreover, a pair of rotation holes 103 into which projections 108a and 108b of the later-described container holding lever 107 are inserted are provided in both side surfaces of the housing 101 so as to be substantially horizontal.

Here, the container 102 in the present embodiment includes: a suction tube 116 through which the detergent contained therein is sucked up; and a tube joint 119 which is provided at an opening surface peripheral portion 117 of the container 102 and connects the suction tube 116 to a pipe 120 of the later-described connection adapter 114.

The projections 108a and 108b which are inserted into the rotation holes 103 so as to be rotatable about the holes 103 are provided to the container holding lever 107. Owing to the pair of the projections 108a and 108b, the container holding lever 107 is mounted on the housing 101 in a state where the container holding lever 107 is rotatable relative to the housing 101.

In addition, the through hole 109 into and out of which a container neck portion 110 of the container 102 comes is provided at substantially the center of the container holding lever 107. When the container 102 is attached within the housing 101 and moved to a predetermined position, a precise position of the container 102 is determined by the container neck portion 110, including a container opening portion 104, entering the hole 109. In attaching the container, a bottom portion of the container holding lever 107 is located at a position lower than (at the body side of the container with respect to) the container opening portion 104 and presses the container 102.

Furthermore, an inclined portion 111 is provided at an end of the container holding lever 107. The inclined portion 111 serves as a guide portion to the housing 101 in attaching the container 102.

The spring 115 is provided within the connection adapter 114. The spring 115 contacts an inner portion of the connection holder 113 at another end thereof and urges the connection adapter 114 downward at all times.

In addition, the pipe 120 which is connected to a pump connection tube 126 connected at one end thereof to the detergent pump 112 is provided within the connection adapter 114. Moreover, similarly to the through hole 109 provided in the connection lever 107, a coupling portion 121 is provided in the connection adapter 114 such that the tube joint 119 and the detergent pump 112 are connected to each other. The coupling portion 121 is provided with a taper angle 121a and structured so as to ensure that the tube joint 119 and the container 102 are concentric with each other in attaching the container 102 to the housing 101.

Furthermore, the connection adapter 114 includes a pair of projection portions 122 which project outward from an outer periphery thereof. The projection portions 122 of the connection adapter 114 are mounted so as to be slidable relative to guides 125 of the connection holder 113. Thus, the connection adapter 114 is slidable up and down within the connection holder 113. An end of each projection portion 122 contacts an upper surface 107a of the container holding lever 107, and another end of each projection portion 122 contacts an inner upper surface of each guide 125 provided in the connection holder 113, when the container holding lever 107 is moved to a highest position thereof in attaching/detaching the container 102, whereby movement of each projection portion 122 is restricted.

The connection holder 113 includes a space 130 in which the connection adapter 114 is stored; a through hole 128 which is located at a center portion thereof and through which the pump connection tube 126 extending from the detergent pump 112 extends to be connected to the pipe 120 of the connection adapter 114; and a pair of the guides 125 which restrict movement of the projection portions 122 of the connection adapter 114. The guides 125 are provided so as to be opposed to each other in a direction substantially perpendicular to a direction in which the container 102 is attached to the housing 101. Thus, rotation of the connection adapter 114 by a pressing force in attaching the container is prevented, and falling-off of the connection adapter 114 is prevented.

By the housing 101, the connection holder 113, and the connection adapter 114 which are described above, in attaching the container 102 to the housing 101, the suction tube 116 and the tube joint 119 of the container 102 and the pipe 120 are positioned concentrically, and communication from the container 102 to the detergent pump 112 is provided.

In storing the container 102 into the storing portion 101a of the housing 101, a mouth portion of the container 102 comes into contact with the inclined portion 111 of the container holding lever 107. Then, when the mouth portion of the container 102 is pressed in along the inclined portion 111, the container holding lever 107 is rotated upward by the pressing force owing to a pair of the projections 108a and 108b to be pressed upward. Here, since the projection portions 122 of the connection adapter 114 contact the upper surface of the container holding lever 107, the connection adapter 114 slides upward within the connection holder 113 in conjunction with the rotation of the container holding lever 107. Then, the spring 115 is compressed upward by the rotation of the container holding lever 107, and the pump connection tube 126 present within the spring 115 is also compressed. In this case, the pump connection tube 126 is compressed in an upper portion of the space 130 of the connection holder 113.

When the mouth portion of the container 102 is further pressed in, the mouth portion is fitted into the hole 109 of the container holding lever 107 and the coupling portion 121 of the connection adapter 114. Thus, by the urging (restoring force) of the spring 115, the connection adapter 114 moves downward and the container holding lever 107 also moves downward via the projection portions 122. Then, by the urging (restoring force) of the spring 115, the connection adapter 114 and the tube joint 119 are liquid-tightly connected to each other.

When the container 102 is set to the housing 101, the container holding lever 107 is held at a higher position as compared to the case where the container 102 is not attached. At that time, the load of the spring 115 is applied also to the container 102 via the connection adapter 114, thereby holding the container 102.

In detaching the container 102, by lifting and rotating the container holding lever 107 upward, it is possible to temporarily release the downward urging of the spring 115 to release the container held state, and thus it is possible to easily take out the container 102 from the detergent container holding portion 100.

As described above, the washing device includes the following invention.

The washing device includes: a washing tank in which a to-be-washed object is washed; a washing water supply portion which supplies washing water to the washing tank; a detergent supply portion which supplies a detergent to the washing tank; a container holding portion which holds a container which communicates with the detergent supply portion and contains the detergent; a washing nozzle which performs washing by scattering the washing water and the detergent to the to-be-washed object within the washing tank; a casing which houses all of these components and includes an opening/closing door through which at least the to-be-washed object is taken in and out; a housing which includes a storing portion which stores the container; a connection holder which is fixed to an upper portion of the storing portion and connected to the detergent supply portion; a lever which is rotatably provided to the housing and presses the container; and a connection adapter which slides up and down between the lever and the connection holder. The connection adapter includes a spring member which is provided therein and contacts the connection holder at one end thereof; and a coupling portion which is coupled to a mouth portion of the container.

According to the above configuration, since the connection adapter slides up and down between the lever and the connection holder and includes therein the spring member which contacts the connection holder at one end thereof, it is possible to fit the mouth portion of the container into the coupling portion and also press a main body of the container against the storing portion via the lever by an elastic force of the spring member. Thus, it is possible to liquid-tightly connect the container to the washing supply portion and maintain this connection state. In addition, it is only necessary to rotate the lever in detaching, and thus the operability is also good.

The lever includes at least a through hole through which the mouth portion of the container is inserted to be guided to the coupling portion, and an inclined surface which is inclined relative to the through hole. Thus, by pressing the container into the storing portion while the mouth portion of the container contacts the inclined surface, it is possible to guide the mouth portion of the container to the coupling portion of the connection adapter and liquid-tightly connect the container to the washing supply portion, and the operability is also further improved.

The connection adapter is stored within the connection holder and includes, on an outer surface thereof, a projection portion which engages the connection holder, and one end of the projection portion contacts an upper surface of the lever. Thus, the elastic force of the spring is transmitted to the lever via the projection portion, and hence it is possible to liquid-tightly connect the container to the washing supply portion only by a lever operation.

At least a pair of the projection portions are provided so as to be opposed to each other, and a direction in which the projection portions are opposed to each other intersects a direction in which the container is inserted into the storing portion. Thus, rotation of the connection adapter when the container is pressed into the storing portion is prevented, and the container and the washing supply portion are liquid-tightly connected to each other.

Finally, possibilities of still another embodiment of the present invention will be described.

In the above-described embodiment, the sliding cover 7 is provided so as to be slidable up and down between the control panel 3 and the handle portion 8, and the sliding cover 7 covers the control panel 3 when the sliding cover 7 is moved from the handle portion 8, thereby physically disabling an operation of the control panel 3. However, a mode of disabling an operation of the control panel 3 is not limited thereto. For example, when the sliding cover opening signal P has been received, the program may be caused to come into the standby state and energization of the setting portion 4 may be turned off, thereby electrically disabling an operation. In addition, a warning message may be displayed on the display portion 5, or a warning sound may be emitted by the sound transmission portion 6, thereby disabling an operation. Furthermore, a vibration mechanism may be provided, thereby disabling an operation. As described above, the mode of "disabling an operation of the control panel 3" also includes a mode in which a visual, auditory, or tactual warning is given to the user and the warning has to be cancelled.

In the above-described embodiment, the micro switches are used as the first and second detection means. However, the present invention is not limited thereto, and various detectors such as a contact type and a non-contact type may be used. In short, a detector that is able to operate in conjunction with opening of a sliding cover from the outside and detect switching to the opened state may be used.

In addition, in the above-described embodiment, the detection means which detects switching of the sliding cover 7 to the opened state is a normally-opened micro switch, and the sliding cover is held so as to contact the micro switch attached at the handle portion side, for determining that the sliding cover returns when the sliding cover is caused to come into the closed state. However, the present invention is not limited thereto, and, for example, detection means which detects switching of the sliding cover 7 to the opened state may be provided to the sliding cover stop mechanism 35, and the micro switch 12 at the handle portion 8 side may be eliminated.

Furthermore, in the above-described embodiment, the sliding cover 7 is slidable up and down. However, the present invention is not limited thereto, and, for example, the sliding cover 7 may be slidable right and left or may be rotatable upward or downward about an upper peripheral portion or a lower peripheral portion thereof, and either one of them may be selected as appropriate in consideration of the opening/closing operability of the opening/closing door 2 or the like. In other words, a mode in which "the cover 7 is movable between the handle portion 8 and the control panel 3" includes a mode in which the user is disabled to physically access either the handle portion 8 or the control panel 3 by the cover 7 covering either the handle portion 8 or the control panel 3.

In the above-described standby mode, when the standby mode is continued for a long period of time, it is conceivable that the detergent or the like sticks to the to-be-washed object to newly add a factor to be washed. Thus, in conjunction with the other control, control may be performed in which, for example, a timer is activated at the time when the standby mode is activated, and re-execution is disabled if a time period until the sliding cover 7 is closed again is equal to or longer than a predetermined time period.

In addition, an accumulated time may be measured from start of an execution mode, and when a predetermined time period or longer has elapsed, the complete stop mode may be activated. When the standby mode is continued for a long period of time or when abnormality occurs in the device itself, shift to the complete stop mode is automatically made, and thus it is possible to safely stop the device.

In the above-described embodiment, the example has been shown in which the sliding cover 7 is manually moved. However, the sliding cover 7 may be electrically moved or mechanically slid based on a switch. In this case, switching to the standby mode at the time when the switch is pressed or at the time when the sliding cover 7 is substantially slightly moved does not deviate from the intention of the present invention at all.

In the above-described embodiment, the example has been shown in which the sliding cover opening signal P is transmitted in order to activate the standby mode. However, the present invention is not limited thereto, and, for example, a sliding cover closing signal P' may be transmitted in a state where it is possible to perform the washing process or the like, namely, in a state where it is not in the standby mode, and the determination section 14 may determine the opened/closed state of the sliding cover on the basis of interruption of the sliding cover closing signal P' when the sliding cover projection portion 11 is separated from the cover micro switch 12 which contacts the sliding cover 7. Furthermore, the execution mode that has been executed before stop is continuously executed again when the sliding cover closing signal P' is detected again.

Similarly, also in the complete stop mode, an opening/closing door closing signal Q' may be transmitted when the opening/closing door 2 is in the closed state, and the determination section 14 may determine that the opening/closing door is in the opened state when the closing signal Q' is interrupted.

In the above-described embodiment, the example has been shown in which the standby mode is activated when abnormality of each of the various sensors is detected. However, control may be performed in which when each of the various sensors detects abnormality, an error message is displayed on the display portion 5 and shift to the complete stop mode is immediately made.

In the drawings of the present embodiment, the container holding portion is provided within the washing device, but the container holding portion may be independent from the washing device and connected to the washing device via a chemical pipe.

As described above, the present invention is not limited to the illustrated embodiment, and it is needless to say that various modifications and changes in design may be made without departing from the gist of the present invention. For example, the positions, the shapes, and the number of the washing nozzles or the various sensors, the type of each pump, and the like are not limited to those in the above-described embodiment.

As described above, in the washing device of the present invention, a door opening/closing mechanism includes: a holding member which holds a door mounted at a front surface portion of a washing device main body, in a closed state; an operation member which is provided within the door and is operated through an operation opening of a front surface of the door from the outside to release the holding by the holding member to make the door openable; a sliding cover which is mounted at the operation opening of the front surface of the door so as to be openable/closable; an operation panel which is provided near the sliding cover and sets washing of the washing device or the like; sliding cover detection means which detects switching of the sliding cover to an opened state and stops spraying of washing water within a washing tank; and door opening/closing detection means which detects switching of the opening/closing door to an opened state and cancels a program being executed. In addition, a detergent supply portion for supplying a detergent from a detergent container includes: a detergent holding portion which is narrowed from an opening portion thereof toward a back thereof, has a narrowest portion having substantially the same width as the detergent container, includes a pressing lever that presses an opening portion and a shoulder portion of the container from above, and sets the detergent container; a detergent supply pump for sucking up a detergent and supplying the detergent to the washing tank; a pipe structure which pipes the detergent from the detergent supply pump to the washing tank; and a spring which urges the pressing lever downward. The pressing lever includes: a joint portion which is connectable to the opening portion of the detergent container; and a supply hose which is connected to the detergent supply pump from the detergent container connected to the joint portion.

In order to open/close the door, it is necessary to initially move the sliding cover such that the sliding cover covers the setting portion, and the user cannot perform an opening/closing operation unless the user slides the sliding cover to above a setting display portion. When performing a slide operation for this, the user is allowed to see a setting operation portion. At that time, the user is allowed to confirm the state of the washing device. In the case where a warning is issued by means of a sound in synchronization with display, it is possible to make visual and auditory confirmation.

It is not possible to open the door unless an operation is performed with the operation member next, and tactual confirmation is required. Therefore, even when the sliding cover is moved by mistake, the time from start of opening of the sliding cover to the time when the door is opened is extended, and spraying of the washing water is stopped before the door is opened. Thus, even when the door is quickly opened, it is possible to prevent the washing water, which is sprayed due to inertia, from scattering to the outside. Furthermore, it is possible to perform, by a continuous motion, operations from pressing of the sliding cover for stopping spraying of the washing water to an operation of the operation member within the door for releasing the holding member, and thus the operability is improved.

Even when a series of these confirmations are quickly performed and the door is opened, since the sliding cover detection mechanism for stopping spraying of the washing water and the like at the time when the sliding cover is caused to come into the opened state and also the door opening/closing detection means for cancelling a washing program when the door is opened are provided, it is possible to open the door without exposing the user to dangers. In addition, since the program being executed such as the washing process is cancelled, washing of the to-be-washed object is restarted from the beginning, and it is possible to guarantee the hygiene of the to-be-washed object.

When the detergent container is pressed in to a predetermined position, the pressing lever is lifted with the pressing-in of the detergent container. Since the pressing lever is urged downward by the spring provided above the pressing lever, when the detergent container is pressed in to a backmost position, the joint portion overlaps the opening portion of the detergent container and is pressed downward by the spring. Since the detergent container storing portion is narrowed toward the back thereof, the detergent container is located at a predetermined position without user's attention.

Therefore, when the detergent container is located at the predetermined position, the pipe is also automatically connected, and when the pressing lever is lifted and the detergent container is pulled out, it is possible to easily take out the container from the washing device. Thus, although time and effort to attach/detach a pipe for sucking up a detergent or a connection portion thereof is generally required in attaching/detaching a container, it is possible to eliminate inconvenience such as inconvenience that the user is forced to perform such a very cumbersome operation, and at that time, the detergent is spilled or the pipe is bent, resulting in influence on a force for sucking up the detergent.

### DESCRIPTION OF THE REFERENCE CHARACTERS

- 1: washing device main body
- 2: opening/closing door
- 3: control panel
- 4: setting portion
- 5: display portion
- 7: sliding cover
- 8: handle portion
- 11: sliding cover projection portion
- 12: cover micro switch
- 13: control unit
- 14: determination section
- 15: memory
- 16: management memory
- 19: washing tank
- 30: casing
- 31: casing micro switch
- 32: holding portion
- 32a: latch lever
- 33: latch
- 35: sliding cover stop mechanism
- 100: container holding portion
- 102: container
- 107: container holding lever
- 113: connection holder
- 114: connection adapter
- 115: spring
- 116: suction tube
- 119: tube joint
- 126: pump connection tube

## Claims

1. A washing device comprising:
a washing tank in which a to-be-washed object is washed;
a washing water supply portion which supplies washing water to the washing tank;
a washing nozzle which performs washing by scattering the washing water to the to-be-washed object within the washing tank; and
a casing which houses all of these components and includes an opening/closing door through which at least the to-be-washed object is taken in and out, wherein
the opening/closing door includes, at a front surface thereof, a control panel which includes at least a setting portion and a display portion, a handle portion for operating a holding portion used in opening/closing the opening/closing door, a cover which covers the handle portion during washing of the to-be-washed object, and first detection means which detects movement of the cover,
the cover is configured to be movable between the handle portion and the control panel, and
by moving the cover during washing of the to-be-washed object, the cover causes the handle portion to appear on the front surface of the opening/closing door to enable an operation of the holding portion and also disables an operation of the control panel by covering the control panel, and the first detection means temporarily stops scattering of the washing water to cause a standby state.

2. A washing device comprising:
a washing tank in which a to-be-washed object is washed;
a washing water supply portion which supplies washing water to the washing tank;
a washing nozzle which performs washing by scattering the washing water to the to-be-washed object within the washing tank; and
a casing which houses all of these components and includes an opening/closing door through which at least the to-be-washed object is taken in and out, wherein
the opening/closing door includes, at a front surface thereof, a control panel which includes at least a setting portion and a display portion, a handle portion for operating a holding portion used in opening/closing the opening/closing door, a cover which covers the handle portion during washing of the to-be-washed object, and first detection means which detects movement of the cover, and
by moving the cover during washing of the to-be-washed object, the cover causes the handle portion to appear on the front surface of the opening/closing door to enable an operation of the holding portion, and the first detection means temporarily stops scattering of the washing water to cause a standby state and disables an operation of the control panel.

3. The washing device according to claim 1 or 2, wherein the handle portion includes second detection means which detects an operation of the holding portion, and the second detection means resets the standby state to cause a complete stop state.

4. The washing device according to claim 2, wherein the first detection means disables an operation of the control panel by at least turning off power of the setting portion.

5. The washing device according to claim 2, wherein the first detection means causes the display portion to display a message of operation disablement to disable an operation of the control panel.

6. The washing device according to any one of claims 1 to 5, wherein a container holding portion which holds a container containing a detergent is provided at a front surface of the casing at which the opening/closing door is opened.

7. The washing device according to any one of claims 1 to 6, wherein the to-be-washed object is a medical instrument.
